# EUROPEAN PATENT SPECIFICATION

(11) **EP 4 023 232 B1**
(45) Date of publication and mention of the grant of the patent: **17.09.2025**
(21) Application number: 19943591.8
(22) Date of filing: 26.08.2019
(51) Int. Cl.: A61K 35/76, A61K 39/395, A61K 31/155, A61P 35/00, A61K 39/00, C07K 14/07

(54) **PHARMACEUTICAL COMPOSITION FOR TREATING CANCER COMPRISING ANTICANCER VIRUS, IMMUNE CHECKPOINT INHIBITOR AND HYDROXYUREA AS ACTIVE INGREDIENTS**
PHARMAZEUTISCHE ZUSAMMENSETZUNG ZUR BEHANDLUNG VON KREBS MIT ANTIKREBSVIRUS, IMMUNCHECKPOINTINHIBITOR UND HYDROXYHARNSTOFF ALS WIRKSTOFFE
COMPOSITION PHARMACEUTIQUE POUR LE TRAITEMENT DU CANCER COMPRENANT UN VIRUS ANTICANCÉREUX, UN INHIBITEUR DE POINT DE CONTRÔLE IMMUNITAIRE ET UNE HYDROXYURÉE EN TANT QUE PRINCIPES ACTIFS

(43) Date of publication of application: 06.07.2022
(73) Proprietor: Bionoxx Inc., Seongnam-si, Gyeonggi-do 13554 (KR)
(72) Inventor: HWANG, Tae-Ho, Seongnam-si, Gyeonggi-do 13554 (KR); CHO, Mong, Seongnam-si, Gyeonggi-do 13554 (KR)
(74) Representative: HGF
(86) International application number: PCT/KR2019/010850
(87) International publication number: WO 2021/040065

(56) References cited:
- WO-A1-2010/135242
- WO-A1-2010/135242
- WO-A1-2011/003194
- WO-A1-2018/195552
- WO-A1-2018/195552
- CA-A1- 3 046 452
- KR-A- 20170 033 364
- KR-A- 20180 099 557
- KR-A- 20190 059 421
- CHRISTOPHERJ LAROCCA ET AL: "Oncolytic viruses and checkpoint inhibitors: combination therapy in clinical trials", CLINICAL AND TRANSLATIONAL MEDICINE, BIOMED CENTRAL LTD, LONDON, UK, vol. 7, no. 1, 14 November 2018 (2018-11-14), pages 1 - 13, XP021262640, DOI: 10.1186/S40169-018-0214-5
- LIU, Z. ET AL: "Rational combination of oncolytic vaccinia virus and PD-L1 blockade works synergistically to enhance therapeutic efficacy", NATURE COMMUNICATIONS, vol. 8, 27 March 2017 (2017-03-27), pages 14754, XP055452350

## Description

### Technical Field

The present invention relates to a pharmaceutical composition for use in treating cancer, comprising, as active ingredients: an oncolytic virus, wherein the oncolytic virus is derived from vaccinia virus, wherein the vaccinia virus is a recombinant virus obtained by deleting the thymidine kinase gene; an immune checkpoint inhibitor; and hydroxyurea.

### Background Art

Oncolytic viruses have excellent tumor-specific targeting ability, proliferation ability in cancer cells, and cancer cell-killing ability. Recently, various clinical studies based on oncolytic viruses have been conducted. In the year 2015, an era of oncolytic virus field began in the US and Europe, as talimogene laherparepvec (T-Vec), which is an oncolytic virus based on herpes simplex virus, was successfully commercialized as a therapeutic agent for advanced melanoma.

Recently, the usefulness of oncolytic viruses exceeds their own efficacy and the viruses activate tumor immunity, thereby showing their potential as a therapeutic agent that is used in combination with another immunotherapeutic agent. Until the year 2000 that was an early stage of development of oncolytic viruses, a direct killing effect of the viruses, which is caused by cancer cell-specific proliferation thereof, was relatively more important. However, subsequent clinical studies have found that the key mechanism of oncolytic viruses was the activation of tumor immunity rather than the direct cancer cell-killing effect. Based on this finding, therapies, in which an oncolytic virus is administered in combination with an immunotherapeutic agent such as an immune checkpoint inhibitor, have recently been developed. It is known that such therapies are possible because the oncolytic virus converts the tumor microenvironment, in which immunity is suppressed, into a tumor microenvironment suitable for immunotherapy.

In a number of clinical studies on vaccinia virus-based oncolytic viruses, oncolytic virus therapy may result in acute tumor necrosis, durable response, or complete response, but in some cases, may lead to a difficult-to-predict result (pharmacodynamics variability) such as progressive disease or early death. For example, for Pexa-vec that is based on a vaccinia virus, in the phase 1 clinical trial, some patients died prematurely within a month after the oncolytic virus therapy and this was associated with persistent systemic inflammatory response and main organs dysfunction. Therefore, in order to enhance the therapeutic effect of an oncolytic virus, it is necessary to understand interactions between cancer cells, the patient's immune status, and the oncolytic virus; and based on this understanding, there is a need for research on techniques capable of increasing clinical efficacy of the oncolytic virus.

### Disclosure of Invention

### Technical Problem

Accordingly, as a resulting of conducting studies to enhance the anticancer effects of oncolytic viruses, the present inventors have found that excellent anticancer effect and safety are obtained in a case where an oncolytic virus, an immune checkpoint inhibitor, and hydroxyurea are co-administered to an individual with cancer, as compared with a conventional case where an oncolytic virus is administered alone, or an oncolytic virus and an immune checkpoint inhibitor are co-administered, thereby completing the present invention.

### Solution to Problem

To achieve the above-mentioned object, in an aspect of the present invention, there is provided a pharmaceutical composition for use in treating cancer, comprising, as active ingredients: an oncolytic virus, wherein the oncolytic virus is derived from vaccinia virus, wherein the vaccinia virus is a recombinant virus obtained by deleting the thymidine kinase gene; an immune checkpoint inhibitor; and hydroxyurea.

In another aspect of the present invention, there is provided a kit for use in treating cancer, comprising: a first composition that comprises an oncolytic virus as an active ingredient, wherein the oncolytic virus is derived from vaccinia virus, wherein the vaccinia virus is a recombinant virus obtained by deleting the thymidine kinase gene; a second composition that comprises hydroxyurea as an active ingredient; and a third composition that comprises an immune checkpoint inhibitor as an active ingredient.

In yet another aspect of the present invention, there is provided an oncolytic virus, an immune checkpoint inhibitor, and hydroxyurea for use in a method for treating cancer, wherein the method comprises administering to an individual with cancer the oncolytic virus, the immune checkpoint inhibitor, and hydroxyurea, wherein the oncolytic virus is derived from vaccinia virus, wherein the vaccinia virus is a recombinant virus obtained by deleting the thymidine kinase gene.

### Advantageous Effects of Invention

The pharmaceutical composition for use in treating cancer, which comprises, as active ingredients, an oncolytic virus, an immune checkpoint inhibitor, and hydroxyurea, of the present invention, wherein the oncolytic virus is derived from vaccinia virus, wherein the vaccinia virus is a recombinant virus obtained by deleting the thymidine kinase gene, has excellent anticancer effect and safety as compared with a conventional case where an oncolytic virus is administered alone or an oncolytic virus and an immune checkpoint inhibitor are co-administered. Accordingly, the pharmaceutical composition, which comprises, as active ingredients, an oncolytic virus, an immune checkpoint inhibitor, and hydroxyurea, of the present invention may be effectively used for the treatment of cancer.

### Brief Description of Drawings

FIG. 1 illustrates results obtained by administering, to mouse renal cancer cell-transplanted mice (Renca), an oncolytic virus (Wyeth VV^{tk-}), a PD-1 inhibitor, and HU, and then measuring tumor volumes on days 0, 4, 10, 14, 17, and 21.
FIG. 2 illustrates results obtained by administering, to mouse renal cancer cell-transplanted mice (Renca), an oncolytic virus (Wyeth VV^{tk-}), a CTLA-4 inhibitor, and HU, and then measuring tumor volumes on days 0, 4, 10, 14, and 17.
FIG. 3 illustrates results obtained by administering, to mouse renal cancer cell-transplanted mice (Renca), an oncolytic virus (Wyeth VV^{tk-}), a PD-L1 inhibitor, and HU, and then measuring tumor volumes on days 0, 4, 10, 14, 17, and 21.
FIG. 4 illustrates results obtained by administering, to mouse breast cancer cell-transplanted mice (4T1), an oncolytic virus (WR VV^{tk-}), a CTLA-4 inhibitor, and HU, and then measuring tumor volumes on days 0, 3, 7, 10, and 14.
FIG. 5 illustrates results obtained by administering, to mouse breast cancer cell-transplanted mice (4T1), an oncolytic virus (WOTS-418), a PD-L1 inhibitor, and HU, and then measuring tumor volumes on days 0, 3, 7, 10, 14, and 18.
FIG. 6 illustrates results obtained by administering, to mouse renal cancer cell-transplanted mice (Renca), a Western Reserve strain vaccinia virus (WR), a CTLA-4 inhibitor, and HU, and then measuring tumor volumes on days 0, 3, and 7.

### Best Mode for Carrying out the Invention

The present invention is defined in the claims.

Hereinafter, the present invention will be described in detail.

In an aspect of the present invention, there is provided a pharmaceutical composition for use in treating cancer, comprising, as active ingredients:
an oncolytic virus, wherein the oncolytic virus is derived from vaccinia virus,
wherein the vaccinia virus is a recombinant virus obtained by deleting the thymidine kinase gene; an immune checkpoint inhibitor; and hydroxyurea.

The oncolytic virus, the immune checkpoint inhibitor, and the hydroxyurea, which are included in the pharmaceutical composition, may be co-administered simultaneously, sequentially, or in reverse order.

Specifically, the oncolytic virus, the immune checkpoint inhibitor, and the hydroxyurea may be administered simultaneously. In addition, the hydroxyurea may be administered first, followed by the immune checkpoint inhibitor, and then the oncolytic virus. The hydroxyurea may be administered first, followed by the oncolytic virus, and then the immune checkpoint inhibitor. The hydroxyurea may be administered first, followed by simultaneous administration of the oncolytic virus and the immune checkpoint inhibitor.

Furthermore, the oncolytic virus may be administered first, followed by the hydroxyurea, and then the immune checkpoint inhibitor. The oncolytic virus may be administered first, followed by the immune checkpoint inhibitor, and then the hydroxyurea. The oncolytic virus may be administered first, followed by simultaneous administration of the hydroxyurea and the immune checkpoint inhibitor.

In addition, the immune checkpoint inhibitor may be administered first, followed by the hydroxyurea, and then the oncolytic virus. The immune checkpoint inhibitor may be administered first, followed by the oncolytic virus, and then the hydroxyurea. The immune checkpoint inhibitor may be administered first, followed by simultaneous administration of the oncolytic virus and the hydroxyurea.

Furthermore, the hydroxyurea may be administered first, followed by the oncolytic virus, followed by the immune checkpoint inhibitor, and then the hydroxyurea again. The hydroxyurea may be administered first, followed by the immune checkpoint inhibitor, followed by the oncolytic virus, and then the hydroxyurea again. The hydroxyurea may be administered first, followed by simultaneous administration of the oncolytic virus and the immune checkpoint inhibitor, and then the hydroxyurea again.

In addition, the hydroxyurea may be administered first, followed by the oncolytic virus, followed by the hydroxyurea again, and then the immune checkpoint inhibitor. The hydroxyurea may be administered first, followed by the immune checkpoint inhibitor, followed by the hydroxyurea again, and then the oncolytic virus.

Furthermore, the hydroxyurea may be administered first, followed by the oncolytic virus, followed by the hydroxyurea again, followed by the immune checkpoint inhibitor, and then the hydroxyurea again. The hydroxyurea may be administered first, followed by the immune checkpoint inhibitor, followed by the hydroxyurea again, followed by the oncolytic virus, and then the hydroxyurea again.

In addition, the oncolytic virus may be administered first, followed by the hydroxyurea, followed by the immune checkpoint inhibitor, and then the hydroxyurea again. The immune checkpoint inhibitor may be administered first, followed by the hydroxyurea, followed by the oncolytic virus, and then the hydroxyurea again.

The oncolytic virus may be derived from adenovirus, herpes simplex virus, measles virus, lentivirus, retrovirus, cytomegalovirus, baculovirus, adeno-associated virus, myxoma virus, vesicular stomatitis virus, poliovirus, Newcastle disease virus, parvovirus, coxsackievirus, senecavirus, vaccinia virus, or orthopoxvirus. Preferably, the oncolytic virus may be derived from vaccinia virus, herpes simplex virus, or adenovirus.

The vaccinia virus may be, but is not limited to, one of the following vaccinia virus strains: Western Reserve (WR), New York vaccinia virus (NYVAC), Wyeth (The New York City Board of Health; NYCBOH), LC16m8, Lister, Copenhagen, Tian Tan, USSR, TashKent, Evans, International Health Division-J (IHD-J), and International Health Division-White (IHD-W). In an embodiment of the present invention, the Western Reserve strain vaccinia virus and the Wyeth strain vaccinia virus were used.

The oncolytic virus may be a wild-type virus or a recombinant virus. Specifically, the recombinant virus may be obtained by deleting at least one gene of the wild-type virus or inserting at least one foreign gene thereinto. Here, the at least one gene of the wild-type virus may be a gene related to viral virulence which encodes any one selected from the group consisting of thymidine kinase (TK), vaccinia growth factor (VGF), WR53.5, F13.5L, F14.5, A56R, B18R, and combinations thereof.

In addition, the at least one foreign gene may be an immune-promoting gene that encodes any one selected from the group consisting of herpes simplex virus thymidine kinase (HSV-TK), HSV-TK variant, granulocyte-macrophage colony-stimulating factor (GM-CSF), granulocyte colony-stimulating factor (G-CSF), cytosine deaminase (CD), carboxylesterase 1, carboxylesterase 2, interferon beta (INF-β), somatostatin receptor 2, and combinations thereof.

Specifically, the recombinant virus may be obtained by deleting TK gene in adenovirus, herpes simplex virus, measles virus, lentivirus, retrovirus, cytomegalovirus, baculovirus, adeno-associated virus, myxoma virus, vesicular stomatitis virus, poliovirus, Newcastle disease virus, parvovirus, coxsackievirus, senecavirus, vaccinia virus, or orthopoxvirus. In an embodiment of the present invention, a recombinant virus obtained by deleting TK gene in a Western Reserve strain vaccinia virus was used, and this recombinant virus was designated as "WR VV^{tk-}". In addition, in an embodiment of the present invention, a recombinant virus obtained by deleting TK gene in a Wyeth strain vaccinia virus was used, and this recombinant virus was designated as "Wyeth VV^{tk-}".

In addition, the recombinant virus may be obtained by deleting TK gene and VGF gene in adenovirus, herpes simplex virus, measles virus, lentivirus, retrovirus, cytomegalovirus, baculovirus, adeno-associated virus, myxoma virus, vesicular stomatitis virus, poliovirus, Newcastle disease virus, parvovirus, coxsackievirus, senecavirus, vaccinia virus, or orthopoxvirus.

Furthermore, the recombinant virus may be obtained by deleting TK gene in and inserting HSV-TK gene into adenovirus, herpes simplex virus, measles virus, lentivirus, retrovirus, cytomegalovirus, baculovirus, adeno-associated virus, myxoma virus, vesicular stomatitis virus, poliovirus, Newcastle disease virus, parvovirus, coxsackievirus, senecavirus, vaccinia virus, or orthopoxvirus.

In addition, the recombinant virus may be obtained by deleting TK gene in and inserting an HSV-TK variant gene into adenovirus, herpes simplex virus, measles virus, lentivirus, retrovirus, cytomegalovirus, baculovirus, adeno-associated virus, myxoma virus, vesicular stomatitis virus, poliovirus, Newcastle disease virus, parvovirus, coxsackievirus, senecavirus, vaccinia virus, or orthopoxvirus. In an embodiment of the present invention, a recombinant virus was used, which is obtained by deleting TK gene in a Western Reserve strain vaccinia virus and inserting, into the deleted position, a gene that is represented by SEQ ID NO: 1 and encodes an HSV-TK variant, and this recombinant virus was designated as "WOTS-418".

Furthermore, the recombinant virus may be obtained by deleting TK gene in and inserting GM-CSF gene into adenovirus, herpes simplex virus, measles virus, lentivirus, retrovirus, cytomegalovirus, baculovirus, adeno-associated virus, myxoma virus, vesicular stomatitis virus, poliovirus, Newcastle disease virus, parvovirus, coxsackievirus, senecavirus, vaccinia virus, or orthopoxvirus.

In addition, the recombinant virus may be obtained by deleting TK gene in and inserting G-CSF gene into adenovirus, herpes simplex virus, measles virus, lentivirus, retrovirus, cytomegalovirus, baculovirus, adeno-associated virus, myxoma virus, vesicular stomatitis virus, poliovirus, Newcastle disease virus, parvovirus, coxsackievirus, senecavirus, vaccinia virus, or orthopoxvirus.

Furthermore, the recombinant virus may be obtained by deleting TK gene in and inserting cytosine deaminase (CD) gene into adenovirus, herpes simplex virus, measles virus, lentivirus, retrovirus, cytomegalovirus, baculovirus, adeno-associated virus, myxoma virus, vesicular stomatitis virus, poliovirus, Newcastle disease virus, parvovirus, coxsackievirus, senecavirus, vaccinia virus, or orthopoxvirus.

In addition, the recombinant virus may be obtained by deleting TK gene in and inserting somatostatin receptor 2 gene into adenovirus, herpes simplex virus, measles virus, lentivirus, retrovirus, cytomegalovirus, baculovirus, adeno-associated virus, myxoma virus, vesicular stomatitis virus, poliovirus, Newcastle disease virus, parvovirus, coxsackievirus, senecavirus, vaccinia virus, or orthopoxvirus.

Furthermore, the recombinant virus may be obtained by deleting TK gene in and inserting any two or more genes selected from the group consisting of genes, each of which encodes herpes simplex virus thymidine kinase (HSV-TK), an HSV-TK variant, granulocyte-macrophage colony-stimulating factor (GM-CSF), granulocyte colony-stimulating factor (G-CSF), cytosine deaminase (CD), or somatostatin receptor 2, into adenovirus, herpes simplex virus, measles virus, lentivirus, retrovirus, cytomegalovirus, baculovirus, adeno-associated virus, myxoma virus, vesicular stomatitis virus, poliovirus, Newcastle disease virus, parvovirus, coxsackievirus, senecavirus, vaccinia virus, or orthopoxvirus.

In addition, the recombinant virus may be obtained by deleting TK gene and VGF gene in and inserting any one gene selected from the group consisting of genes, each of which encodes HSV-TK, an HSV-TK variant, GM-CSF, G-CSF, CD, or somatostatin receptor 2, and combinations thereof, into adenovirus, herpes simplex virus, measles virus, lentivirus, retrovirus, cytomegalovirus, baculovirus, adeno-associated virus, myxoma virus, vesicular stomatitis virus, poliovirus, Newcastle disease virus, parvovirus, coxsackievirus, senecavirus, vaccinia virus, or orthopoxvirus.

As used herein, the term "gene deletion" means that a gene is not expressed due to partial or complete deletion of the gene, or insertion of a foreign gene thereinto. In a case where partial deletion occurs in the gene, some amino acids at the N-terminus or C-terminus of a polypeptide expressed by the gene may be deleted.

As used herein, the term "thymidine kinase (TK)" refers to an enzyme that is called thymidine kinase and involved in nucleotide biosynthesis. The TK is an enzyme used for nucleotide biosynthesis in both cells and viruses. Here, for the cells, normal cells do not divide anymore, and thus no TK exists therein; and even for rapidly dividing cells such as hair follicle cells, TK is not present in an amount sufficient for viruses to utilize. From these viewpoints, a virus is allowed to proliferate only in the presence of cancer cells, in which TK is present, by deletion of TK gene therein, so that the cancer cells may be selectively killed.

As used herein, the term "vaccinia growth factor (VGF)" refers to a polypeptide that has sequence homology to epidermal growth factor and stimulates cell proliferation around infected cells. A vaccinia virus replicates better in proliferating cells, and thus may be advantageously used for viral replication *in vivo.* In order to cause an oncolytic virus to proliferate more specifically only in cancer cells, the virus may additionally undergo deletion of VGF gene in addition to deletion of the TK gene.

As used herein, the term "GM-CSF", which is called granulocyte-macrophage colony-stimulating factor, refers to a protein secreted by macrophages, T cells, mast cells, natural killer cells, endothelial cells, and fibroblasts. GM-CSF stimulates stem cells to produce granulocytes (neutrophils, basophils, eosinophils) and monocytes. In addition, GM-CSF rapidly increases the number of macrophages, thereby inducing an immune response. The GM-CSF may be of human origin and may be a protein having the sequence of GenBank: AAA52578.1.

As used herein, the term "CD", which is called cytosine deaminase, refers to an enzyme that catalyzes hydrolytic deamination of cytosine into uracil and ammonia.

As used herein, the term "G-CSF", which is called granulocyte colony-stimulating factor, refers to a cytokine produced by macrophages, fibroblasts, endothelial cells, and the like upon stimulation by inflammation or endotoxin. The G-CSF promotes production of neutrophils. The G-CSF may be of human origin (rhGCSF) and may be a protein having the sequence of GenBank: AAA03056.1.

As used herein, the term "somatostatin receptor 2" refers to a protein encoded by SSTR2 gene in humans. The somatostatin receptor 2 is expressed mainly in tumors, and patients with neuroendocrine tumors, who overexpress somatostatin receptor 2, show improved prognosis. The somatostatin receptor 2 has capacity to stimulate apoptosis in many cells, including cancer cells.

As used herein, the term "hydroxyurea" refers to a compound having the following formula.

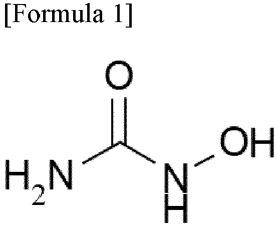

The hydroxyurea is known as an anticancer agent that inhibits DNA synthesis; however, the exact mechanism thereof is not elucidated. In addition, the hydroxyurea may be included in the pharmaceutical composition in the form of a commercialized drug that contains hydroxyurea. Examples of the commercialized drug that contains hydroxyurea may include, but are not limited to, Hydroxyurea^{®}, Hydrea^{®}, Droxia^{™}, Mylocel^{™}, Siklos^{®}, and Hydrine^{®} capsule. The hydroxyurea may be taken orally, and parenteral administration thereof is also possible.

A dosage of the oncolytic virus varies depending on the individual's condition and body weight, the severity of disease, the type of drug, the route and period of administration, and may be appropriately selected by a person skilled in the art. The dosage may be such that a patient receives a vaccinia virus at 1×10⁵ to 1×10¹⁸ of virus particles, infectious virus units (TCID₅₀), or plaque forming units (pfu). Specifically, the dosage may be such that a patient receives an oncolytic virus at 1×10⁵, 2×10⁵, 5×10⁵, 1×10⁶, 2×10⁶, 5×10⁶, 1×10⁷, 2×10⁷, 5×10⁷, 1×10⁸, 2×10⁸, 5×10⁸, 1×10⁹, 2×10⁹, 5×10⁹, 1×10¹⁰, 5×10¹⁰, 1×10¹¹, 5×10¹¹, 1×10¹², 1×10¹³, 1×10¹⁴, 1×10¹⁵, 1×10¹⁶, 1×10¹⁷, or higher of virus particles, infectious virus units, or plaque forming units, and various numerical values and ranges between the above-mentioned numerical values may also be included therein. Preferably, the oncolytic virus may be administered at a dose of 1×10⁵ to 1×10¹⁰ pfu. More preferably, the oncolytic virus may be administered at a dose of equal to or greater than 1×10⁵ and lower than 1×10⁹ pfu. In an embodiment of the present invention, the oncolytic virus was administered at 1×10⁵ or ×10⁷ pfu.

In addition, the hydroxyurea may be administered at a dose of 1 mg/kg/day to 100 mg/kg/day, or 10 mg/kg/day to 90 mg/kg/day. Specifically, the hydroxyurea may be administered at a dose of 10 mg/kg/day to 90 mg/kg/day, 15 mg/kg/day to 80 mg/kg/day, 20 mg/kg/day to 70 mg/kg /day, 25 mg/kg/day to 65 mg/kg/day, or 30 mg/kg/day to 60 mg/kg/day. In an embodiment of the present invention, the hydroxyurea was administered at 30 mg/kg/day or 60 mg/kg/day. Depending on the dosage, the hydroxyurea may be administered in divided doses several times a day. Specifically, the hydroxyurea may be administered 1 to 4 times a day or 1 to 2 times a day.

The immune checkpoint inhibitor refers to a substance that inhibits the mechanism of cancer cells which interferes with activation of T cells, and may be any one selected from the group consisting of anti-PD-L1 antibody, anti-PD-1 antibody, anti-CTLA4 antibody, anti-PD-L2 antibody, LTF2 control antibody, anti-LAG3 antibody, anti-A2aR antibody, anti-TIGIT antibody, anti-TIM-3 antibody, anti-B7-H3 antibody, anti-B7-H4 antibody, anti-VISTA antibody, anti-CD47 antibody, anti-BTLA antibody, anti-KIR antibody, anti-IDO antibody, and combinations thereof.

Cancer cells hijack the immune checkpoint system as a mechanism to evade immune responses. Specifically, cancer cells use immune checkpoint receptors to evade immune responses, and representative examples of the immune checkpoint receptor include PD-L1, PD-1, CTLA-4, and the like. In order to prevent these cancer cells from evading immunity, an immune checkpoint inhibitor, which is a molecule that specifically binds to an immune checkpoint receptor, is used for the treatment of cancer. The first immune checkpoint inhibitor was ipilimumab (Yervoy^{®}), which is a monoclonal antibody that specifically binds to cytotoxic T-lymphocyte associated antigen-4 (CTLA-4). The next developed immune checkpoint therapeutic agents were monoclonal antibodies against programmed cell death-1 (PD-1) and a ligand thereof, that is, programmed death ligand-1 (PD-L1). As representative drugs, anti-PD-1 antibodies such as nivolumab (Opdivo^{®}) and pembrolizumab (Keytruda^{®}), and anti-PD-L1 antibodies such as avelumab (Bavencio^{®}), atezolizumab (Tecentriq^{®}), and durvalumab (Imfinzi^{®}) may be mentioned.

In addition, research has been conducted on monoclonal antibodies that specifically bind to various immune checkpoint receptors such as glucocorticoid-induced TNFR-related protein (GITR), killer cell immunoglobulin-like receptor (KIR), lymphocyte-activation gene-3 (LAG-3), T-cell immunoglobulin and mucin-domain containing-3 (TIM-3), and tumor-necrosis factor receptor superfamily member 4 (TNFRSF4).

Administration of the immune checkpoint inhibitor may be performed in compliance with the usage and dose set by each manufacturer. The immune checkpoint inhibitor may be administered at a dose of 0.1 mg/kg to 10 mg/kg, or 1 mg/kg to 5 mg/kg. For example, for Opdivo Injection. that contains nivolumab as an active ingredient, 3 mg/kg may be administered via intravenous instillation over 60 minutes at 2-week intervals; and the usage and dose thereof as a combination therapy may be such that 1 mg/kg is administered via intravenous instillation over 30 minutes. In addition, for Keytruda Injection. that contains pembrolizumab as an active ingredient, 200 mg may be administered via intravenous instillation over 30 minutes at 3-week intervals. As such, even with the same anti-PD-1 antibody, the usage and dose thereof vary depending on the product. Thus, administration thereof is preferably performed in compliance with the usage and dose set by each manufacturer.

The cancer may be solid cancer or blood cancer. Specifically, the blood cancer may be any one selected from the group consisting of lymphoma, acute leukemia, and multiple myeloma. The solid cancer may be any one selected from the group consisting of lung cancer, colorectal cancer, prostate cancer, thyroid cancer, breast cancer, brain cancer, head and neck cancer, esophageal cancer, skin cancer, thymic cancer, gastric cancer, colon cancer, liver cancer, ovarian cancer, uterine cancer, bladder cancer, rectal cancer, gallbladder cancer, biliary tract cancer, pancreatic cancer, and combinations thereof.

In addition, the pharmaceutical composition of the present invention may further comprise a physiologically acceptable carrier. In addition, the pharmaceutical composition of the present invention may further comprise suitable excipients and diluents commonly used in the preparation of pharmaceutical compositions. In addition, the pharmaceutical composition may be formulated in the form of an injection according to a conventional method.

In a case of being formulated as preparations for parenteral administration, the pharmaceutical composition may be formulated into sterilized aqueous solutions, non-aqueous solutions, suspensions, emulsions, freeze-dried preparations, suppositories, or the like. For the non-aqueous solution or the suspension, propylene glycol, polyethylene glycol, vegetable oil such as olive oil, injectable ester such as ethyl oleate, or the like may be used. As the base of the suppository, Witepsol^{™}, macrogol, Tween^{™} 61, cacao butter, laurin fat, glycerogelatin, or the like may be used.

Regarding the administration route, dosage, and frequency of administration, the pharmaceutical composition may be administered to a subject in a variety of ways and amounts depending on the patient's condition and the presence or absence of side effects; and the optimal administration route, dosage, and frequency of administration therefor may be selected by a person skilled in the art within a suitable range. In addition, the pharmaceutical composition may be administered in combination with another drug or physiologically active substance whose therapeutic effect is known for the disease to be treated, or may be formulated in the form of a combination preparation with the other drug.

The pharmaceutical composition may be administered parenterally, and such administration may be performed by any suitable method, such as intratumoral, intraperitoneal, subcutaneous, intradermal, intranodal, intravenous, or intraarterial administration. Among these, intratumoral, intraperitoneal, or intravenous administration may be preferred. On the other hand, the dosage of the pharmaceutical composition may be determined depending on the administration schedule, the total dosage, and the patient's health condition.

In another aspect of the present invention, there is provided a kit for treating cancer, comprising a first composition that includes an oncolytic virus as an active ingredient, a second composition that includes hydroxyurea as an active ingredient, and a third composition that includes an immune checkpoint inhibitor as an active ingredient.

The oncolytic virus, the immune checkpoint inhibitor, and the hydroxyurea are as described above for the pharmaceutical composition.

A dosage of the first composition varies depending on the individual's condition and body weight, the severity of disease, the type of drug, the route and period of administration, and may be appropriately selected by a person skilled in the art. The dosage may be such that a patient receives a vaccinia virus at 1×10⁵ to 1×10¹⁸ of virus particles, infectious virus units (TCID₅₀), or plaque forming units (pfu). Specifically, the dosage may be such that a patient receives an oncolytic virus at 1×10⁵, 2×10⁵, 5×10⁵, 1×10⁶, 2×10⁶, 5×10⁶, 1×10⁷, 2×10⁷, 5×10⁷, 1×10⁸, 2×10⁸, 5×10⁸, 1×10⁹, 2×10⁹, 5×10⁹, 1×10¹⁰, 5×10¹⁰, 1×10¹¹, 5×10¹¹, 1×10¹², 1×10¹³, 1×10¹⁴, 1×10¹⁵, 1×10¹⁶ , 1×10¹⁷, or higher of virus particles, infectious virus units, or plaque forming units, and various numerical values and ranges between the above-mentioned numerical values may also be included therein. Preferably, the oncolytic virus may be administered at a dose of 1×10⁵ to 1×10¹⁰ pfu. More preferably, the oncolytic virus may be administered at a dose of equal to or greater than 1×10⁵ and lower than 1×10⁹ pfu. In an embodiment of the present invention, the first composition was administered at 1×10⁵ or ×10⁷ pfu.

In addition, the second composition may be administered at a dose of 1 mg/kg/day to 100 mg/kg/day, or 10 mg/kg/day to 90 mg/kg/day. Specifically, the second composition may be administered at a dose of 10 mg/kg/day to 90 mg/kg/day, 15 mg/kg/day to 80 mg/kg/day, 20 mg/kg/day to 70 mg/kg /day, 25 mg/kg/day to 65 mg/kg/day, or 30 mg/kg/day to 60 mg/kg/day. In an embodiment of the present invention, the second composition was administered at 30 mg/kg/day or 60 mg/kg/day. Depending on the dosage, the second composition may be administered in divided doses several times a day. Specifically, the second composition may be administered 1 to 4 times a day or 1 to 2 times a day.

Administration of the third composition may be performed in compliance with the usage and dose of an immune checkpoint inhibitor included in the third composition, which are set by each manufacturer. The dosage of the third composition may be 0.1 mg/kg to 10 mg/kg, or 1 mg/kg to 5 mg/kg.

The cancer may be solid cancer or blood cancer. Specifically, the blood cancer may be any one selected from the group consisting of lymphoma, acute leukemia, and multiple myeloma. The solid cancer may be any one selected from the group consisting of lung cancer, colorectal cancer, prostate cancer, thyroid cancer, breast cancer, brain cancer, head and neck cancer, esophageal cancer, skin cancer, thymic cancer, gastric cancer, colon cancer, liver cancer, ovarian cancer, uterine cancer, bladder cancer, rectal cancer, gallbladder cancer, biliary tract cancer, pancreatic cancer, and combinations thereof.

The first composition, the second composition, and the third composition may further comprise a physiologically acceptable carrier. In addition, the pharmaceutical composition of the present invention may further comprise suitable excipients and diluents commonly used in the preparation of pharmaceutical compositions. In addition, the pharmaceutical composition may be formulated in the form of an injection according to a conventional method.

In a case of being formulated as preparations for parenteral administration, the first composition, the second composition, and the third composition may be formulated into sterilized aqueous solutions, non-aqueous solutions, suspensions, emulsions, freeze-dried preparations, suppositories, or the like. For the non-aqueous solution or the suspension, propylene glycol, polyethylene glycol, vegetable oil such as olive oil, injectable ester such as ethyl oleate, or the like may be used. As the base of the suppository, Witepsol^{™}, macrogol, Tween^{™} 61, cacao butter, laurin fat, glycerogelatin, or the like may be used.

Regarding the administration route, total dosage, and frequency of administration, the first composition, the second composition, and the third composition may be administered to a subject in a variety of ways and amounts depending on the patient's condition and the presence or absence of side effects; and the optimal administration route, dosage, and frequency of administration therefor may be selected by a person skilled in the art within a suitable range. In addition, the pharmaceutical composition may be administered in combination with another drug or physiologically active substance whose therapeutic effect is known for the disease to be treated, or may be formulated in the form of a combination preparation with the other drug.

The first composition, the second composition, and the third composition may be administered parenterally, and such administration may be performed by any suitable method, such as intratumoral, intraperitoneal, subcutaneous, intradermal, intranodal, intravenous, or intraarterial administration. Among these, intratumoral, intraperitoneal, or mtravenous administration may be preferred. On the other hand, dosages of the first composition, the second composition, and the third composition may be determined depending on the administration schedule, the total dosage, and the patient's health condition.

In addition, the first composition may be administered twice to an individual, and the administration may be performed at intervals of 7 to 30 days. Specifically, the first composition may be administered at intervals of 7 days, 14 days, 21 days, or 30 days.

The second composition may be administered before or after administration of the first composition. Specifically, the second composition may be continuously administered once a day starting from 3 to 5 days before administration of the first composition, and may be continuously administered once a day for 9 to 28 days starting from within 24 hours of or after 24 hours of administration of the first composition. In an embodiment of the present invention, the second composition may be continuously administered once a day starting from 1 to 3 days before administration of the first composition, and may be administered once a day for 13 days, 17 days, 18 days, or 28 days after administration of the first composition.

The third composition may be continuously administered at least once a week for 1 to 10 weeks after administration of the first composition. Specifically, the third composition may be administered continuously at least twice a week for 1 to 8 weeks after administration of the first composition.

In yet another aspect of the present invention, there is provided an oncolytic virus, an immune checkpoint inhibitor, and hydroxyurea for use in a method for treating cancer, wherein the method comprises administering to an individual with cancer the oncolytic virus, the immune checkpoint inhibitor, and hydroxyurea, wherein the oncolytic virus is derived from vaccinia virus, wherein the vaccinia virus is a recombinant virus obtained by deleting the thymidine kinase gene.

The oncolytic virus, the immune checkpoint inhibitor, and the hydroxyurea are as described above for the pharmaceutical composition.

The oncolytic virus, the immune checkpoint inhibitor, and the hydroxyurea may be co-administered simultaneously, sequentially, or in reverse order. Specifically, the oncolytic virus, the immune checkpoint inhibitor, and the hydroxyurea may be administered simultaneously. In addition, the hydroxyurea may be administered first, followed by the immune checkpoint inhibitor, and then the oncolytic virus. The hydroxyurea may be administered first, followed by the oncolytic virus, and then the immune checkpoint inhibitor. The hydroxyurea may be administered first, followed by simultaneous administration of the oncolytic virus and the immune checkpoint inhibitor.

Furthermore, the oncolytic virus may be administered first, followed by the hydroxyurea, and then the immune checkpoint inhibitor. The oncolytic virus may be administered first, followed by the immune checkpoint inhibitor, and then the hydroxyurea. The oncolytic virus may be administered first, followed by simultaneous administration of the hydroxyurea and the immune checkpoint inhibitor.

In addition, the immune checkpoint inhibitor may be administered first, followed by the hydroxyurea, and then the oncolytic virus. The immune checkpoint inhibitor may be administered first, followed by the oncolytic virus, and then the hydroxyurea. The immune checkpoint inhibitor may be administered first, followed by simultaneous administration of the oncolytic virus and the hydroxyurea.

Furthermore, the hydroxyurea may be administered first, followed by the oncolytic virus, followed by the immune checkpoint inhibitor, and then the hydroxyurea again. The hydroxyurea may be administered first, followed by the immune checkpoint inhibitor, followed by the oncolytic virus, and then the hydroxyurea again. The hydroxyurea may be administered first, followed by simultaneous administration of the oncolytic virus and the immune checkpoint inhibitor, and then the hydroxyurea again.

In addition, the hydroxyurea may be administered first, followed by the oncolytic virus, followed by the hydroxyurea again, and then the immune checkpoint inhibitor. The hydroxyurea may be administered first, followed by the immune checkpoint inhibitor, followed by the hydroxyurea again, and then the oncolytic virus.

Furthermore, the hydroxyurea may be administered first, followed by the oncolytic virus, followed by the hydroxyurea again, followed by the immune checkpoint inhibitor, and then the hydroxyurea again. The hydroxyurea may be administered first, followed by the immune checkpoint inhibitor, followed by the hydroxyurea again, followed by the oncolytic virus, and then the hydroxyurea again.

In addition, the oncolytic virus may be administered first, followed by the hydroxyurea, followed by the immune checkpoint inhibitor, and then the hydroxyurea again. The immune checkpoint inhibitor may be administered first, followed by the hydroxyurea, followed by the oncolytic virus, and then the hydroxyurea again.

A dosage of the oncolytic virus varies depending on the individual's condition and body weight, the severity of disease, the type of drug, the route and period of administration, and may be appropriately selected by a person skilled in the art. The dosage may be such that a patient receives a vaccinia virus at 1×10⁵ to 1×10¹⁸ of virus particles, infectious virus units (TCID₅₀), or plaque forming units (pfu). Specifically, the dosage may be such that a patient receives an oncolytic virus at 1×10⁵, 2×10⁵, 5×10⁵, 1×10⁶, 2×10⁶, 5×10⁶, 1×10⁷, 2×10⁷, 5×10⁷, 1×10⁸, 2×10⁸, 5×10⁸, 1×10⁹, 2×10⁹, 5×10⁹, 1×10¹⁰, 5×10¹⁰, 1×10¹¹, 5×10¹¹, 1×10¹², 1×10¹³, 1×10¹⁴, 1×10¹⁵, 1×10¹⁶ , 1×10¹⁷, or higher of virus particles, infectious virus units, or plaque forming units, and various numerical values and ranges between the above-mentioned numerical values may also be included therein. Preferably, the oncolytic virus may be administered at a dose of 1×10⁵ to 1×10¹⁰ pfu. More preferably, the oncolytic virus may be administered at a dose of equal to or greater than 1×10⁵ and lower than 1×10⁹ pfu. In an embodiment of the present invention, the oncolytic virus was administered at 1×10⁵ or ×10⁷ pfu.

In addition, the hydroxyurea may be administered at a dose of 1 mg/kg/day to 100 mg/kg/day, or 10 mg/kg/day to 90 mg/kg/day. Specifically, the hydroxyurea may be administered at a dose of 10 mg/kg/day to 90 mg/kg/day, 15 mg/kg/day to 80 mg/kg/day, 20 mg/kg/day to 70 mg/kg /day, 25 mg/kg/day to 65 mg/kg/day, or 30 mg/kg/day to 60 mg/kg/day. In an embodiment of the present invention, the hydroxyurea was administered at 30 mg/kg/day or 60 mg/kg/day. Depending on the dosage, the hydroxyurea may be administered in divided doses several times a day. Specifically, the hydroxyurea may be administered 1 to 4 times a day or 1 to 2 times a day.

Administration of the immune checkpoint inhibitor may be performed in compliance with the usage and dose set by each manufacturer. The immune checkpoint inhibitor may be administered at a dose of 0.1 mg/kg to 10 mg/kg, or 1 mg/kg to 5 mg/kg. For example, for Opdivo Injection. that contains nivolumab as an active ingredient, 3 mg/kg may be administered via intravenous instillation over 60 minutes at 2-week intervals; and the usage and dose thereof as a combination therapy may be such that 1 mg/kg is administered via intravenous instillation over 30 minutes. In addition, for Keytruda Injection. that contains pembrolizumab as an active ingredient, 200 mg may be administered via intravenous instillation over 30 minutes at 3-week intervals. As such, even with the same anti-PD-1 antibody, the usage and dose thereof vary depending on the product. Thus, administration thereof is preferably performed in compliance with the usage and dose set by each manufacturer.

In addition, the oncolytic virus may be administered 1 to 10 times or 2 to 5 times, and may be administered to an individual at intervals of 7 to 30 days. Specifically, the oncolytic virus may be administered at intervals of 7 days, 14 days, 21 days, or 30 days.

The hydroxyurea may be administered before, during, or after administration of the oncolytic virus. Specifically, the hydroxyurea may be administered before or after administration of the oncolytic virus. The hydroxyurea may be continuously administered once a day starting from 3 to 5 days before administration of the oncolytic virus, and may be continuously administered once a day for 9 to 28 days starting from within 24 hours of or after 24 hours of administration of the oncolytic virus. In an embodiment of the present invention, the hydroxyurea may be continuously administered once a day starting from 1 to 3 days before administration of the oncolytic virus, and may be administered once a day for 13 days, 17 days, 18 days, or 28 days after administration of the oncolytic virus.

The immune checkpoint inhibitor may be administered before, during, or after administration of the oncolytic virus. Specifically, the immune checkpoint inhibitor may be administered after administration of the oncolytic virus. The immune checkpoint inhibitor may be continuously administered at least once a week for 1 to 10 weeks after administration of the oncolytic virus. Specifically, the immune checkpoint inhibitor may be administered continuously at least twice a week for 1 to 8 weeks after administration of the oncolytic virus.

The cancer may be solid cancer or blood cancer. Specifically, the blood cancer may be any one selected from the group consisting of lymphoma, acute leukemia, and multiple myeloma. The solid cancer may be any one selected from the group consisting of lung cancer, colorectal cancer, prostate cancer, thyroid cancer, breast cancer, brain cancer, head and neck cancer, esophageal cancer, skin cancer, thymic cancer, gastric cancer, colon cancer, liver cancer, ovarian cancer, uterine cancer, bladder cancer, rectal cancer, gallbladder cancer, biliary tract cancer, pancreatic cancer, and combinations thereof.

The hydroxyurea may be administered orally or parenterally. Specifically, the hydroxyurea may be administered parenterally, and such administration may be performed intraperitoneally or intravenously.

In addition, the immune checkpoint inhibitor may be administered intraperitoneally or intravenously.

The oncolytic virus may be administered parenterally, and such administration may be performed by any suitable method, such as intratumoral, intraperitoneal, subcutaneous, intradermal, intranodal, intravenous, or intraarterial administration. Among these, intratumoral, intraperitoneal, or intravenous administration may be preferred. Meanwhile, the dosages of the oncolytic virus, the immune checkpoint inhibitor, and the hydroxyurea may be determined according to the administration schedule, the dosage, and the patient's health condition.

As used herein, the term "individual" refers to a person who has or is suffering from a disease in a state that may be alleviated, inhibited, or treated by administering the pharmaceutical composition of the present invention.

As used herein, the term "administration" means introducing an effective amount of a substance into an individual by an appropriate method, and administration of the vaccinia virus and the hydroxyurea may be performed via a common route that allows the substances to reach a target tissue.

In addition, the oncolytic virus, the immune checkpoint inhibitor, and the hydroxyurea may be administered in combination with another drug or physiologically active substance whose therapeutic effect is known for the disease to be treated, or may be formulated in the form of a combination preparation with the other drug.

### Mode for the Invention

Hereinafter, the present invention will be described in more detail by way of examples. However, the following examples are for illustrative purposes only, and the scope of the present invention is not limited thereto.

### Preparation Example 1. Production of oncolytic viruses (Wyeth VV^{tk-}, WR VV^{tk-})

### Preparation Example 1.1. Construction of shuttle plasmid vector

To produce oncolytic viruses in which thymidine kinase (TK) gene is deleted, the wild-type vaccinia viruses, that is, Wyeth strain (NYC Department of Health) and Western Reserve strain were purchased from the American Type Culture Collection (ATCC). For recombination, a TK region in the wild-type vaccinia virus was subjected to substitution using a shuttle plasmid vector that contains firefly luciferase reporter (p7.5 promoter) gene or GFP gene.

### Preparation Example 1.2. Production of oncolytic viruses

To obtain oncolytic viruses, HeLa cells (ATCC) were seeded in 6-well plates at 4×10⁵ cells per well, and then culture was performed in EMEM medium containing 10% fetal bovine serum. Subsequently, treatment with the wild-type vaccinia virus was performed at an MOI of 0.05. 2 hours later, the medium was replaced with EMEM medium containing 2% fetal bovine serum, and then the cells were transfected with 4 µg of the shuttle plasmid vector, which was constructed in Preparation Example 1.1 and linearized, using Xfect^{™} polymer (Clonetech 631317, USA). Culture was performed for 4 hours. Subsequently, the medium was replaced with EMEM medium containing 2% fetal bovine serum, and then culture was performed for 72 hours. Finally, the infected cells were collected, and then freezing and thawing were repeated 3 times. Subsequently, the cells were lysed by sonication, and a sucrose cushion method was used to obtain free oncolytic viruses, which were designated Wyeth VV^{tk-} or WR VV^{tk-}.

### Preparation Example 2. Production of oncolytic virus (WOTS-418)

To produce an oncolytic virus in which thymidine kinase (TK) gene is deleted and which expresses a mutated herpes simplex virus thymidine kinase (HSV1-TK) gene, a TK region in the Western Reserve strain wild-type vaccinia virus was subjected to substitution using (as a shuttle vector) pUC57amp+ plasmid (Genewiz, USA) into which synthesized mutated type 1 HSV-TK gene (pSE/L promoter) of SEQ ID NO: 1 and firefly luciferase reporter (p7.5 promoter) gene were recombined. An oncolytic virus was obtained in the same manner as in Preparation Example 1.2 using the shuttle vector as constructed above, and this oncolytic virus was designated WOTS-418.

### Experimental Example 1. Identification of anticancer effect of oncolytic virus (Wyeth VV^{tk-}), PD-1 inhibitor, and hydroxyurea in mouse renal cancer cell-transplanted mice: Renca (I)

To identify an additional effect caused by administration of hydroxyurea upon co-administration of an oncolytic virus and a PD-1 inhibitor (CD279, BioXCell), which is one of the immune checkpoint inhibitors, an experiment was conducted using mouse renal cancer cell-transplanted mice.

First, Balb/c mice (female, 8-week-old) purchased from ORIENT BIO (Busan, Korea) were subjected to a one-week acclimatization period, and then allografted with Renca cancer cell line (Korea Cell Line Bank) at 5×10⁶ cells. The tumor volume was observed until it reached 200 mm³ to 300 mm³, and then oncolytic virus (Wyeth VV^{tk-}) administration was started. The oncolytic virus has limited proliferation in an allograft model.

The produced mouse renal cancer cell-transplanted mice were divided into 5 groups (n=5). The group receiving intraperitoneal administration of saline was set as a negative control group, the group receiving the mouse PD-1 inhibitor, the group receiving intratumoral administration of the oncolytic virus (Wyeth VV^{tk-}, 1×10⁷ pfu), and the group receiving co-administration of the oncolytic virus (Wyeth VV^{tk-}, 1×10⁷ pfu) and the PD-1 inhibitor were set as positive control groups. In addition, the group receiving co-administration of the oncolytic virus (Wyeth VV^{tk-}, 1×10⁷ pfu), the PD-1 inhibitor, and hydroxyurea (30 mg/kg) was set as an experimental group. Here, the oncolytic virus was intratumorally administered once; the PD-1 inhibitor was intraperitoneally administered once every 2 days on days 14, 16, 18, and 20; and the hydroxyurea was intraperitoneally administered 6 times per week.

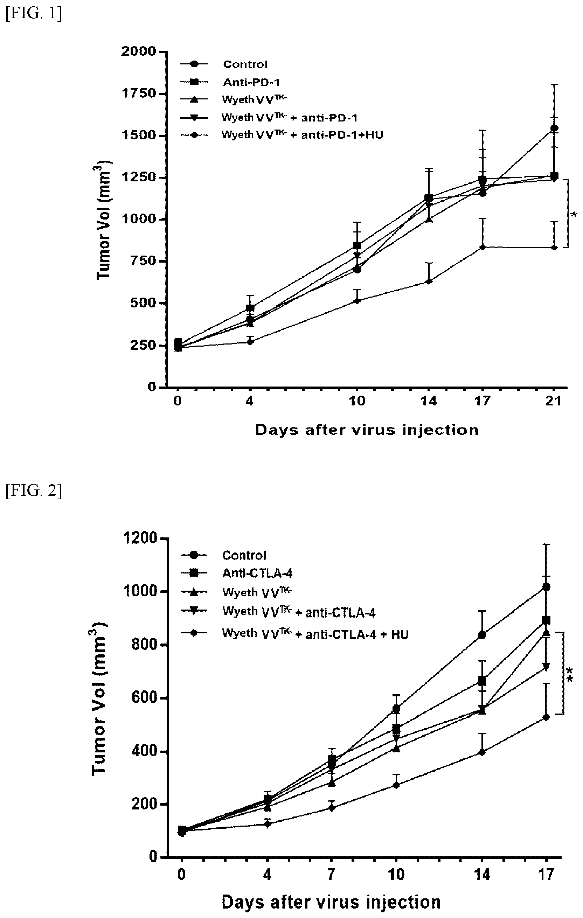

Tumor volumes were measured on days 0, 4, 10, 14, 17, and 21 after the drug administration to the mice of each group. As a result, it was identified that the tumor volume in the mice of the experimental group was significantly suppressed as compared with the tumor volume in the mice of the positive control groups (FIG. 1).

### Experimental Example 2. Identification of anticancer effect of oncolytic virus (Wyeth VV^{tk-}), CTLA-4 inhibitor, and hydroxyurea in mouse renal cancer cell-transplanted mice: Renca (II)

To identify an additional effect caused by administration of hydroxyurea upon co-administration of an oncolytic virus and a CTLA-4 inhibitor (B7-H1, BioXCell), which is an immune checkpoint inhibitor, an experiment was conducted using mouse renal cancer cell-transplanted mice.

First, Balb/c mice (female, 8-week-old) purchased from ORIENT BIO (Busan, Korea) were subjected to a one-week acclimatization period, and then allografted with Renca cancer cell line (Korea Cell Line Bank) at 5×10⁶ cells. The tumor volume was observed until it reached 50 mm³ to 150 mm³, and then oncolytic virus (Wyeth VV^{tk-}) administration was started. The oncolytic virus has limited proliferation in an allograft model.

The produced mouse renal cancer cell-transplanted mice were divided into 5 groups (n=6). The group receiving intraperitoneal administration of saline was set as a negative control group, the group receiving the CTLA-4 inhibitor, the group receiving intratumoral administration of the oncolytic virus (Wyeth VV^{tk-}, 1×10⁷ pfu), and the group receiving co-administration of the oncolytic virus (Wyeth VV^{tk-}, 1×10⁷ pfu) and the CTLA-4 inhibitor were set as positive control groups. In addition, the group receiving co-administration of the oncolytic virus (Wyeth VV^{tk-}, 1×10⁷ pfu), the CTLA-4 inhibitor, and hydroxyurea (30 mg/kg) was set as an experimental group. Here, the oncolytic virus was intratumorally administered once; the CTLA-4 inhibitor was intraperitoneally administered once every 2 days on days 3, 5, 7, and 9; and the hydroxyurea was intraperitoneally administered 6 times per week.

Tumor volumes were measured on days 0, 4, 7, 10, 14, and 17 after the drug administration to the mice of each group. As a result, it was identified that the tumor volume in the mice of the experimental group was significantly suppressed as compared with the tumor volume in the mice of the positive control groups (FIG. 2). From these results, it was identified that an excellent mouse renal cancer inhibition effect was exhibited in a case where hydroxyurea is also administered upon co-administration of an oncolytic virus and an immune checkpoint inhibitor (CTLA-4 inhibitor).

### Experimental Example 3. Identification of anticancer effect of oncolytic virus (Wyeth VV^{tk-}), PD-L1 inhibitor, and hydroxyurea in mouse renal cancer cell-transplanted mice: Renca (III)

To identify an additional effect caused by administration of hydroxyurea upon co-administration of an oncolytic virus and a PD-L1 inhibitor (CD152, BioXCell), which is one of the immune checkpoint inhibitors, an experiment was conducted using mouse renal cancer cell-transplanted mice.

First, Balb/c mice (female, 8-week-old) purchased from ORIENT BIO (Busan, Korea) were subjected to a one-week acclimatization period, and then allografted with Renca cancer cell line (Korea Cell Line Bank) at 5×10⁶ cells. The tumor volume was observed until it reached 50 mm³ to 100 mm³, and then oncolytic virus (Wyeth VV^{tk-}) administration was started. The oncolytic virus has limited proliferation in an allograft model.

The produced mouse renal cancer cell-transplanted mice were divided into 5 groups (n=6). The group receiving intraperitoneal administration of saline was set as a negative control group, the group receiving the PD-L1 inhibitor (300 µg/mouse), the group receiving intratumoral administration of the oncolytic virus (Wyeth VV^{tk-}, 1×10⁷ pfu), and the group receiving co-administration of the oncolytic virus (Wyeth VV^{tk-}, 1×10⁷ pfu) and the PD-L1 inhibitor were set as positive control groups. In addition, the group receiving co-administration of the oncolytic virus (Wyeth VV^{tk-}, 1×10⁷ pfu), the PD-L1 inhibitor, and hydroxyurea (30 mg/kg) was set as an experimental group. Here, the oncolytic virus was intratumorally administered once; the PD-L1 inhibitor was intraperitoneally administered on days 0, 3, 7, 10, 14, 17, and 21; and the hydroxyurea was intraperitoneally administered 6 times per week.

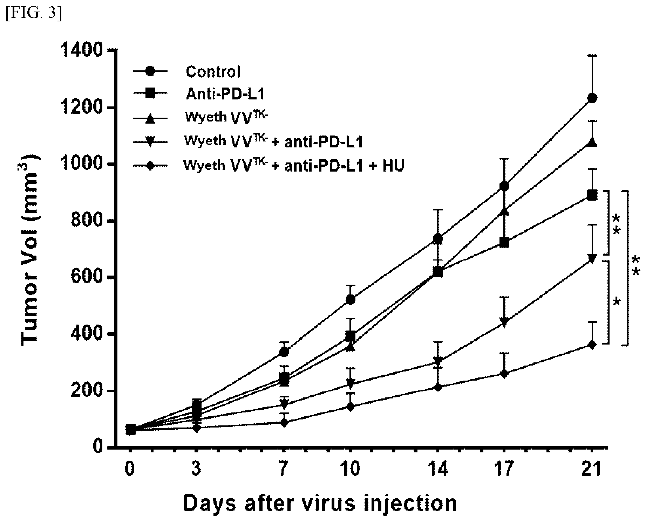

Tumor volumes were measured on days 0, 3, 7, 10, 14, 17, and 21 after the drug administration to the mice of each group. As a result, it was identified that the tumor volume in the mice of the experimental group was significantly suppressed as compared with the tumor volume in the mice of the positive control groups (FIG. 3). In particular, it was identified that in a case where comparison was made on the tumor volume before mouse sacrifice, the experimental group exhibited the tumor volume that is about 46% smaller than the group having received co-administration of the oncolytic virus and the PD-L1 inhibitor.

From these results, it was identified that an excellent mouse renal cancer inhibition effect was exhibited in a case where hydroxyurea is also administered upon co-administration of an oncolytic virus and an immune checkpoint inhibitor (PD-L1 inhibitor).

### Experimental Example 4. Identification of anticancer effect of oncolytic virus (WR VV^{tk-}), CTLA-4 inhibitor, and hydroxyurea in mouse breast cancer cell-transplanted mice: 4T1 (I)

### Experimental Example 4.1. Production of mouse breast cancer cell-transplanted mice and drug administration

To identify an additional effect caused by administration of hydroxyurea upon co-administration of an oncolytic virus and a CTLA-4 inhibitor (B7-H1, BioXCell), an experiment was conducted using mouse breast cancer cell-transplanted mice.

First, Balb/c mice (female, 8-week-old) purchased from ORIENT BIO (Busan, Korea) were subjected to a one-week acclimatization period, and then allografted with 4T1 cancer cell line (Korea Cell Line Bank) at 1×10⁶ cells. The tumor volume was observed until it reached 50 mm³ to 150 mm³, and then oncolytic virus (WR VV^{tk-}) administration was started. The Western Reserve strain vaccinia virus-derived oncolytic virus (WR VV^{tk-}) has stronger proliferative capacity in an allograft model than Wyeth strain vaccinia virus-derived oncolytic virus.

The produced mouse breast cancer cell-transplanted mice were divided into 5 groups (n=5). The group receiving intraperitoneal administration of saline was set as a negative control group, the group receiving the CTLA-4 inhibitor (300 µg/mouse), the group receiving intratumoral administration of the oncolytic virus (WR VV^{tk-}, 1×10⁷ pfu), and the group receiving co-administration of the oncolytic virus (WR VV^{tk-}, 1×10⁷ pfu) and the CTLA-4 inhibitor were set as positive control groups. In addition, the group receiving co-administration of the oncolytic virus (WR VV^{tk-}, 1×10⁷ pfu), the CTLA-4 inhibitor, and hydroxyurea (30 mg/kg) was set as an experimental group. Here, the oncolytic virus was intratumorally administered twice; the CTLA-4 inhibitor was intraperitoneally administered on days 3, 5, 7, and 9; and the hydroxyurea was intraperitoneally administered 6 times per week.

### Experimental Example 4.2. Checking for changes in tumor volume

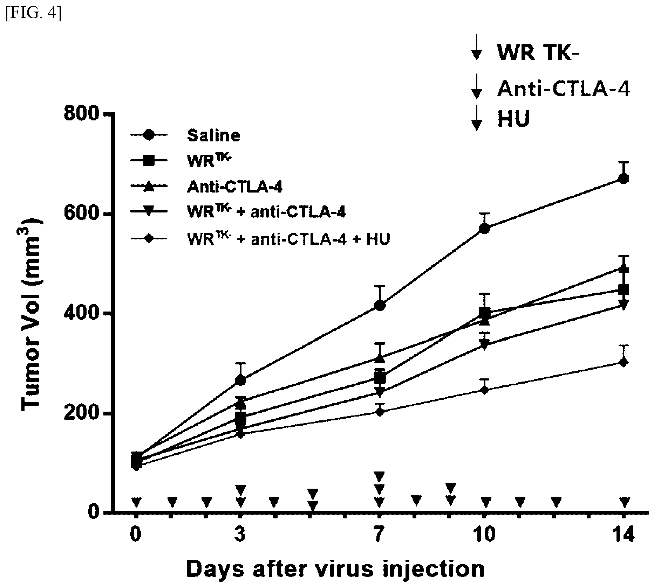

Tumor volumes were measured on days 0, 3, 7, 10, and 14 after the drug administration to the mice of each group. As a result, it was identified that the tumor volume in the mice of the experimental group was significantly suppressed as compared with the tumor volume in the mice of the positive control groups (FIG. 4).

### Experimental Example 4.3. Analysis of survival

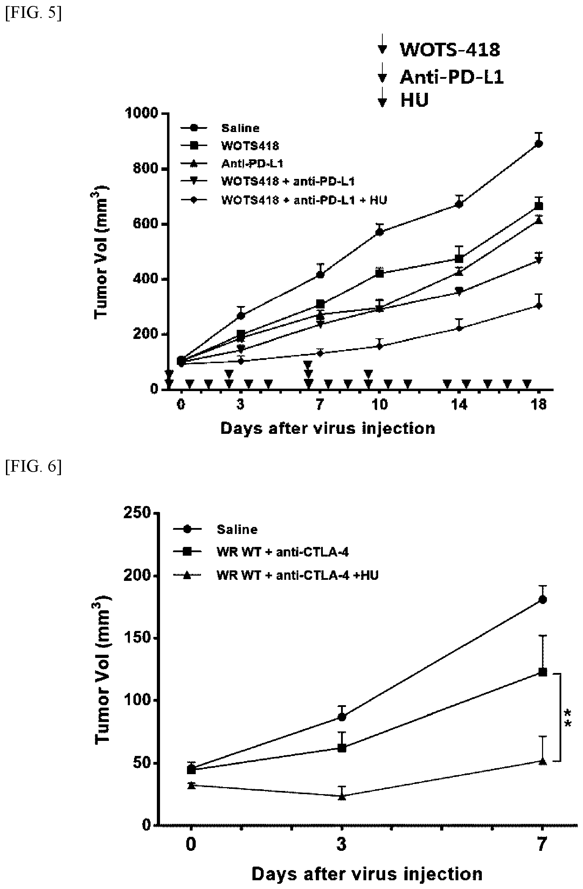

In addition, it was identified that as for survival, the mice of the experimental group exhibited the best survival period and survival rate (FIG. 5). From these results, it was identified that a significant effect was exhibited in a case where hydroxyurea is also administered upon co-administration of an oncolytic virus and a CTLA-4 inhibitor to mouse breast cancer cell-transplanted mice.

### Experimental Example 5. Identification of anticancer effect of oncolytic virus (WOTS-418), PD-L1 inhibitor, and hydroxyurea in mouse breast cancer cell-transplanted mice: 4T1 (II)

### Experimental Example 5.1. Production of mouse breast cancer cell-transplanted mice and drug administration

To identify an additional effect caused by administration of hydroxyurea upon co-administration of an oncolytic virus and a PD-L1 inhibitor (CD152, BioXCell), an experiment was conducted using mouse breast cancer cell-transplanted mice.

First, Balb/c mice (female, 8-week-old) purchased from ORIENT BIO (Busan, Korea) were subjected to a one-week acclimatization period, and then allografted with 4T1 cancer cell line (Korea Cell Line Bank) at 1×10⁶ cells. The tumor volume was observed until it reached 50 mm³ to 100 mm³, and then Western Reserve strain vaccinia virus-derived oncolytic virus (WOTS-418) administration was started. The Western Reserve strain has stronger proliferative capacity in an allograft model than the Wyeth strain.

The produced mouse breast cancer cell-transplanted mice were divided into 5 groups (n=6). The group receiving intraperitoneal administration of saline was set as a negative control group, the group receiving the PD-L1 inhibitor (300 µg/mouse), the group receiving intratumoral administration of the oncolytic virus (WOTS-418, 1×10⁷ pfu), and the group receiving co-administration of the oncolytic virus (WOTS-418, 1×10⁷ pfu) and the PD-L1 inhibitor were set as positive control groups. In addition, the group receiving co-administration of the oncolytic virus (WOTS-418, 1×10⁷ pfu), the PD-L1 inhibitor, and hydroxyurea (30 mg/kg) was set as an experimental group. Here, the oncolytic virus was intratumorally administered twice; the PD-L1 inhibitor was intraperitoneally administered on days 3, 5, 7, and 9; and the hydroxyurea was intraperitoneally administered 6 times per week.

### Experimental Example 5.2. Checking for changes in tumor volume

Tumor volumes were measured on days 0, 3, 7, 10, and 14 after the drug administration to the mice of each group in Experimental Example 5.1. As a result, it was identified that the tumor volume in the mice of the experimental group was significantly suppressed as compared with the tumor volume in the mice of the positive control groups (FIG. 5). In particular, it was identified that in a case where comparison was made on the tumor volume before mouse sacrifice, the experimental group exhibited the tumor volume that is about 30% smaller than the group having received co-administration of the oncolytic virus and the PD-L1 inhibitor.

From these results, it was identified that a synergistic effect was exhibited in suppressing mouse breast cancer in a case where hydroxyurea is also administered upon co-administration of an oncolytic virus and an immune checkpoint inhibitor (PD-L1 inhibitor).

### Experimental Example 5.3. Analysis of survival

A 30-day survival rate was analyzed for the mice of each group in Experimental Example 5.1. As a result, it was identified that the mice of the experimental group had a higher survival rate than that in the mice of the negative and positive control groups.

### Experimental Example 6. Analysis of survival for oncolytic virus (WR, WOTS-418), PD-L1 inhibitor, and hydroxyurea in mouse colorectal cancer cell-transplanted mice: CT-26 I

To identify the safety upon co-administration of Western Reserve strain vaccinia virus (WR), a PD-L1 inhibitor, and hydroxyurea, a survival period was analyzed using mouse colorectal cancer cell-transplanted mice.

First, Balb/c mice purchased from ORIENT BIO (Busan, Korea) were subjected to a one-week acclimatization period, and then subcutaneously transplanted with a mouse colorectal cancer (CT-26) cell line (Korea Cell Line Bank) at 1×10⁶ cells. After 7 days, the oncolytic virus (WR) and the PD-L1 inhibitor were intraperitoneally administered, and hydroxyurea was administered daily for 5 days from the next day. On the other hand, the Western Reserve strain vaccinia virus has stronger proliferative capacity in an allograft model than Wyeth strain vaccinia virus.

The produced mouse colorectal cancer cell-transplanted mice were divided into 5 groups (n=13). The group receiving intraperitoneal administration of saline was set as a negative control group, the group receiving the PD-L1 inhibitor (200 µg/mouse) alone, and the group receiving co-administration of the oncolytic virus (WOTS-418) and hydroxyurea (30 mg/kg) were set as positive control groups. In addition, the group receiving co-administration of the oncolytic virus (WR, 1×10⁶ pfu; or WOTS-418, 1×10⁷ pfu), the PD-L1 inhibitor, and hydroxyurea was set as an experimental group. Here, the oncolytic virus was intratumorally administered once; the PD-L1 inhibitor was intraperitoneally administered on days 1, 4, 8, and 11; and the hydroxyurea was intraperitoneally administered 5 times per week.

Survival curves for the mice of each group were analyzed. As a result, it was observed that the mice of the experimental group had the longest survival period as compared with the mice of the negative and positive control groups. From these results, it was identified that safety was improved in a case of co-administration of an oncolytic virus, an immune checkpoint inhibitor, and hydroxyurea.

### Experimental Example 7. Analysis of survival with Western Reserve Strain vaccinia virus (WR), CTLA-4 Inhibitor, and hydroxyurea in mouse renal cancer cell-transplanted mice: Renca (IV)

To identify an additional effect caused by administration of hydroxyurea upon co-administration of Western Reserve Strain vaccinia virus and a CTLA-4 inhibitor (B7-H1, BioXCell), which is one of the immune checkpoint inhibitors, an experiment was conducted using mouse renal cancer cell-transplanted mice.

First, Balb/c mice (female, 8-week-old) purchased from ORIENT BIO (Busan, Korea) were subjected to a one-week acclimatization period, and then allografted with Renca cancer cell line (Korea Cell Line Bank) at 5×10⁶ cells. The tumor volume was observed until it reached 30 mm³ to 50 mm³, and then Western Reserve strain vaccinia virus (WR) administration was started. The Western Reserve strain vaccinia virus has stronger proliferative capacity in an allograft model than Wyeth strain vaccinia virus.

The produced mouse renal cancer cell-transplanted mice were divided into 4 groups (n=4). The group receiving intraperitoneal administration of saline was set as a negative control group, the group receiving co-administration of the Western Reserve strain vaccinia virus (WR, 1×10⁵ pfu) and hydroxyurea (30 mg/kg), and the group receiving co-administration of the Western Reserve strain vaccinia virus and the CTLA-4 inhibitor (150 µg/mouse) were set as positive control groups. In addition, the group receiving administration of the Western Reserve strain vaccinia virus, the CTLA-4 inhibitor, and hydroxyurea was set as an experimental group. Here, the Western Reserve strain vaccinia virus was intratumorally administered once; the CTLA-4 inhibitor was intraperitoneally administered on days 2, 4. 6, and 8; and the hydroxyurea was intraperitoneally administered 4 times per week.

Tumor volumes were measured on days 0, 3, and 7 after the drug administration to the mice of each group. As a result, it was identified that the tumor volume in the mice of the experimental group was significantly suppressed as compared with the tumor volume in the mice of the positive control groups (FIG. 6).

## Claims

1. A pharmaceutical composition for use in treating cancer, comprising as active ingredients:
an oncolytic virus, wherein the oncolytic virus is derived from vaccinia virus, wherein the vaccinia virus is a recombinant virus obtained by deleting the thymidine kinase gene;
an immune checkpoint inhibitor; and
hydroxyurea.

2. The pharmaceutical composition for use according to claim 1, wherein the vaccinia virus is one of the following vaccinia virus strains: Western Reserve (WR), New York vaccinia virus (NYVAC), Wyeth (The New York City Board of Health; NYCBOH), LC16m8, Lister, Copenhagen, Tian Tan, USSR, TashKent, Evans, International Health Division-J (IHD-J), and International Health Division-White (IHD-W).

3. The pharmaceutical composition for use according to claim 1, wherein the oncolytic virus is obtained by further deleting at least one gene in the wild-type virus or inserting at least one foreign gene thereinto, optionally
wherein the at least one gene in wild-type virus is any one selected from the group consisting of vaccinia growth factor gene, WR53.5 gene, F13.5L gene, F14.5 gene, A56R gene, B18R gene, and combinations thereof, or
wherein the at least one foreign gene is a gene that encodes herpes simplex virus thymidine kinase (HSV-TK), an HSV-TK variant, granulocyte-macrophage colony-stimulating factor (GM-CSF), cytosine deaminase (CD), carboxylesterase 1, carboxylesterase 2, interferon beta (INF-β), granulocyte colony-stimulating factor (G-CSF), or somatostatin receptor 2.

4. The pharmaceutical composition for use according to claim 1, wherein the immune checkpoint inhibitor is any one selected from the group consisting of anti-PD-L1 antibody, anti-PD-1 antibody, anti-CTLA-4 antibody, anti-PD-L2 antibody, LTF2 control antibody, anti-LAG3 antibody, anti-A2aR antibody, antiTIGIT antibody, anti-TIM-3 antibody, anti-B7-H3 antibody, anti-B7-H4 antibody, anti-VISTA antibody, anti-CD47 antibody, anti-BTLA antibody, antiKIR antibody, anti-IDO antibody, and combinations thereof.

5. The pharmaceutical composition for use according to claim 1, wherein the cancer is any one selected from the group consisting of lung cancer, colorectal cancer, prostate cancer, thyroid cancer, breast cancer, brain cancer, head and neck cancer, esophageal cancer, skin cancer, thymic cancer, gastric cancer, colon cancer, liver cancer, ovarian cancer, uterine cancer, bladder cancer, rectal cancer, gallbladder cancer, biliary tract cancer, pancreatic cancer, and combinations thereof.

6. A kit for use in treating cancer, comprising:
a first composition that comprises an oncolytic virus as an active ingredient, wherein the oncolytic virus is derived from vaccinia virus, wherein the vaccinia virus is a recombinant virus obtained by deleting the thymidine kinase gene;
a second composition that comprises hydroxyurea as an active ingredient; and
a third composition that comprises an immune checkpoint inhibitor as an active ingredient.

7. An oncolytic virus, an immune checkpoint inhibitor, and hydroxyurea for use in a method for treating cancer, wherein the method comprises administering to an individual with cancer the oncolytic virus, the immune checkpoint inhibitor, and hydroxyurea, wherein the oncolytic virus is derived from vaccinia virus, wherein the vaccinia virus is a recombinant virus obtained by deleting the thymidine kinase gene.

8. The oncolytic virus, the immune checkpoint inhibitor, and hydroxyurea for use according to claim 7, wherein the oncolytic virus, the immune checkpoint inhibitor, and the hydroxyurea are co-administered simultaneously, sequentially, or in reverse order, or
wherein the hydroxyurea is administered before, during, or after administration of the oncolytic virus.

9. The oncolytic virus, the immune checkpoint inhibitor, and hydroxyurea for use according to claim 7, wherein the hydroxyurea is administered 3 to 5 days before administration of the oncolytic virus and is continuously administered once a day for 9 to 28 days after administration of the oncolytic virus.

10. The oncolytic virus, the immune checkpoint inhibitor, and hydroxyurea for use according to claim 7, wherein the immune checkpoint inhibitor is administered after administration of the oncolytic virus.

11. The oncolytic virus, the immune checkpoint inhibitor, and hydroxyurea for use according to claim 7, wherein the immune checkpoint inhibitor is continuously administered at least once a week for 1 week to 10 weeks after administration of the oncolytic virus.

12. The oncolytic virus, the immune checkpoint inhibitor, and hydroxyurea for use according to claim 7, wherein the hydroxyurea is administered at a dose of 10 mg/kg/day to 90 mg/kg/day, or wherein the oncolytic virus is administered at a dose of 1x10⁵ pfu to 1x10¹⁰ pfu.

13. The oncolytic virus, the immune checkpoint inhibitor, and hydroxyurea for use according to claim 7, wherein the oncolytic virus is administered to the individual at intervals of 7 to 30 days.

14. The oncolytic virus, the immune checkpoint inhibitor, and hydroxyurea for use according to claim 7, wherein the hydroxyurea is administered intraperitoneally or intravenously, or
wherein the oncolytic virus is administered intratumorally, intraperitoneally, or intravenously.

## Patentansprüche

1. Pharmazeutische Zusammensetzung zur Verwendung bei der Behandlung von Krebs, umfassend als Wirkstoffe:
ein onkolytisches Virus, wobei das onkolytische Virus von Vaccinia-Virus stammt, wobei das Vaccinia-Virus ein rekombinantes Virus ist, das durch Deletieren des Thymidinkinase-Gens erhalten wird;
einen Immuncheckpointinhibitor; und
Hydroxyharnstoff.

2. Pharmazeutische Zusammensetzung zur Verwendung nach Anspruch 1, wobei das Vaccinia-Virus einer der folgenden Vaccinia-Virusstämme ist: Western Reserve (WR), New York Vaccinia Virus (NYVAC), Wyeth (The New York City Board of Health; NYCBOH), LC16m8, Lister, Kopenhagen, Tian Tan, USSR, TashKent, Evans, International Health Division-J (IHD-J) und International Health Division-White (IHD-).

3. Pharmazeutische Zusammensetzung zur Verwendung nach Anspruch 1, wobei das onkolytische Virus durch weiteres Deletieren von zumindest einem Gen in dem Wildtyp-Virus oder Einfügen von zumindest einem Fremdgen darin erhalten wird,
wobei optional das zumindest eine Gen in Wildtyp-Virus ein beliebiges ausgewählt aus der Gruppe bestehend aus Vaccinia-Wachstumsfaktor-Gen, WR53.5-Gen, F13.5L-Gen, F14.5-Gen, A56R-Gen, B18R-Gen und Kombinationen davon ist, oder
wobei das zumindest eine Fremdgen ein Gen ist, das Herpes-simplex-Virus-Thymidinkinase (HSV-TK), eine HSV-TK-Variante, Granulozyten-Makrophagen-Kolonie-stimulierenden Faktor (GM-CSF), Cytosin-Deaminase (CD), Carboxylesterase 1, Carboxylesterase 2, Interferon beta (INF-β), Granulozyten-Kolonie-stimulierenden Faktor (G-CSF) oder Somatostatin-Rezeptor 2 kodiert.

4. Pharmazeutische Zusammensetzung zur Verwendung nach Anspruch 1, wobei der Immuncheckpointinhibitor ein beliebiger ausgewählt aus der Gruppe bestehend aus Anti-PD-L1-Antikörper, Anti-PD-1-Antikörper, Anti-CTLA-4-Antikörper, Anti-PD-L2-Antikörper, LTF2-Kontrollantikörper, Anti-LAG3-Antikörper, Anti-A2aR-Antikörper, AntiTIGIT-Antikörper, Anti-TIM-3-Antikörper, Anti-B7-H3-Antikörper, Anti-B7-H4-Antikörper, Anti-VISTA-Antikörper, Anti-CD47-Antikörper, Anti-BTLA-Antikörper, Anti-KIR-Antikörper, Anti-IDO-Antikörper und Kombinationen davon ist.

5. Pharmazeutische Zusammensetzung zur Verwendung nach Anspruch 1, wobei der Krebs ein beliebiger ausgewählt aus der Gruppe bestehend aus Lungenkrebs, Kolorektalkrebs, Prostatakrebs, Schilddrüsenkrebs, Brustkrebs, Hirnkrebs, Kopf- und Halskrebs, Speiseröhrenkrebs, Hautkrebs, Thymuskrebs, Magenkrebs, Darmkrebs, Leberkrebs, Eierstockkrebs, Gebärmutterkrebs, Blasenkrebs, Rektalkrebs, Gallenblasenkrebs, Gallengangskrebs, Bauchspeicheldrüsenkrebs und Kombinationen davon ist.

6. Kit zur Verwendung bei der Behandlung von Krebs, umfassend:
eine erste Zusammensetzung, die ein onkolytisches Virus als Wirkstoff umfasst, wobei das onkolytische Virus von Vaccinia-Virus stammt, wobei das Vaccinia-Virus ein rekombinantes Virus ist, das durch Deletieren des Thymidinkinase-Gens erhalten wird;
eine zweite Zusammensetzung, die Hydroxyharnstoff als Wirkstoff umfasst; und
eine dritte Zusammensetzung, die einen Immuncheckpointinhibitor als Wirkstoff umfasst.

7. Onkolytisches Virus, Immuncheckpointinhibitor und Hydroxyharnstoff zur Verwendung in einem Verfahren zur Behandlung von Krebs, wobei das Verfahren Verabreichen des onkolytischen Virus, des Immuncheckpointinhibitors und des Hydroxyharnstoffs an ein Individuum mit Krebs umfasst, wobei das onkolytische Virus von Vaccinia-Virus stammt, wobei das Vaccinia-Virus ein rekombinantes Virus ist, das durch Deletieren des Thymidinkinase-Gens erhalten wird.

8. Onkolytisches Virus, Immuncheckpointinhibitor und Hydroxyharnstoff zur Verwendung nach Anspruch 7, wobei das onkolytische Virus, der Immuncheckpointinhibitor und der Hydroxyharnstoff gleichzeitig, sequenziell oder in umgekehrter Reihenfolge co-verabreicht werden, oder
wobei der Hydroxyharnstoff vor, während oder nach Verabreichung des onkolytischen Virus verabreicht wird.

9. Onkolytisches Virus, Immuncheckpointinhibitor und Hydroxyharnstoff zur Verwendung nach Anspruch 7, wobei der Hydroxyharnstoff 3 bis 5 Tage vor Verabreichung des onkolytischen Virus verabreicht wird und einmal am Tag für 9 bis 28 Tage nach Verabreichung des onkolytischen Virus kontinuierlich verabreicht wird.

10. Onkolytisches Virus, Immuncheckpointinhibitor und Hydroxyharnstoff zur Verwendung nach Anspruch 7, wobei der Immuncheckpointinhibitor nach Verabreichung des onkolytischen Virus verabreicht wird.

11. Onkolytisches Virus, Immuncheckpointinhibitor und Hydroxyharnstoff zur Verwendung nach Anspruch 7, wobei der Immuncheckpointinhibitor zumindest einmal pro Woche für 1 Woche bis 10 Wochen nach Verabreichung des onkolytischen Virus kontinuierlich verabreicht wird.

12. Onkolytisches Virus, Immuncheckpointinhibitor und Hydroxyharnstoff zur Verwendung nach Anspruch 7, wobei der Hydroxyharnstoff in einer Dosis von 10 mg/kg/Tag bis 90 mg/kg/Tag verabreicht wird, oder wobei das onkolytische Virus in einer Dosis von 1x10⁵ pfu bis 1x10¹⁰ pfu verabreicht wird.

13. Onkolytisches Virus, Immuncheckpointinhibitor und Hydroxyharnstoff zur Verwendung nach Anspruch 7, wobei das onkolytische Virus dem Individuum in Intervallen von 7 bis 30 Tagen verabreicht wird.

14. Onkolytisches Virus, Immuncheckpointinhibitor und Hydroxyharnstoff zur Verwendung nach Anspruch 7, wobei der Hydroxyharnstoff intraperitoneal oder intravenös verabreicht wird, oder
wobei das onkolytische Virus intratumoral, intraperitoneal oder intravenös verabreicht wird.

## Revendications

1. Composition pharmaceutique destinée à être utilisée dans le traitement d'un cancer, comprenant en tant qu'ingrédients actifs :
un virus oncolytique, ledit virus oncolytique étant dérivé du virus de la vaccine, ledit virus de la vaccine étant un virus recombinant obtenu par délétion du gène de la thymidine kinase ;
un inhibiteur de point de contrôle immunitaire ; et
l'hydroxyurée.

2. Composition pharmaceutique destinée à être utilisée selon la revendication 1, dans laquelle le virus de la vaccine est l'une des souches de virus de la vaccine suivantes : Réserve de l'Ouest (Western Reserve, WR), le virus de la vaccine de New York (NYVAC), Wyeth (The New York City Board of Health ; NYCBOH), LC16m8, Lister, Copenhagen, Tian Tan, URSS, TashKent, Evans, International Health Division-J (IHD-J) et International Health Division-White (IHD-).

3. Composition pharmaceutique destinée à être utilisée selon la revendication 1, dans laquelle le virus oncolytique est obtenu en délétant en outre au moins un gène du virus de type sauvage ou en y insérant au moins un gène étranger, éventuellement
dans laquelle l'au moins un gène du virus de type sauvage est l'un quelconque choisi dans le groupe constitué par le gène du facteur de croissance de la vaccine, le gène WR53.5, le gène F13.5L, le gène F14.5, le gène A56R, le gène B18R et des combinaisons de ceux-ci, ou
dans laquelle l'au moins un gène étranger est un gène qui code pour la thymidine kinase du virus de l'herpès simplex (HSV-TK), une variante de HSV-TK, le facteur de stimulation des colonies de granulocytes et de macrophages (GM-CSF), la cytosine désaminase (CD), la carboxylestérase 1, la carboxylestérase 2, l'interféron bêta (INF-β), le facteur de stimulation des colonies de granulocytes (G-CSF) ou le récepteur de la somatostatine 2.

4. Composition pharmaceutique destinée à être utilisée selon la revendication 1, dans laquelle l'inhibiteur de point de contrôle immunitaire est l'un quelconque choisi dans le groupe constitué par un anticorps anti-PD-L1, un anticorps anti-PD-1, un anticorps anti-CTLA-4, un anticorps anti-PD-L2, un anticorps de contrôle LTF2, un anticorps anti LAG3, un anticorps anti-A2aR, un anticorps anti-TIGIT, un anticorps anti-TIM-3, un anticorps anti-B7-H3, un anticorps anti-B7-H4, un anticorps anti-VISTA, un anticorps anti-CD47, un anticorps anti-BTLA, un anticorps anti-KIR, un anticorps anti-IDO, et des combinaisons de ceux-ci.

5. Composition pharmaceutique destinée à être utilisée selon la revendication 1, ledit cancer étant l'un quelconque choisi dans le groupe constitué par le cancer du poumon, le cancer colorectal, le cancer de la prostate, le cancer de la thyroïde, le cancer du sein, le cancer du cerveau, le cancer de la tête et du cou, le cancer de l'œsophage, le cancer de la peau, le cancer thymique, le cancer de l'estomac, le cancer du côlon, le cancer du foie, le cancer de l'ovaire, le cancer de l'utérus, le cancer de la vessie, le cancer du rectum, le cancer de la vésicule biliaire, le cancer des voies biliaires, le cancer du pancréas et des combinaisons de ceux-ci.

6. Kit destiné à être utilisé dans le traitement d'un cancer, comprenant :
une première composition qui comprend un virus oncolytique en tant qu'ingrédient actif, ledit virus oncolytique étant dérivé du virus de la vaccine, ledit virus de la vaccine étant un virus recombinant obtenu par délétion du gène de la thymidine kinase ;
une deuxième composition qui comprend de l'hydroxyurée en tant qu'ingrédient actif ; et
une troisième composition qui comprend un inhibiteur de point de contrôle immunitaire en tant qu'ingrédient actif.

7. Virus oncolytique, inhibiteur de point de contrôle immunitaire et hydroxyurée destinés à être utilisés dans un procédé de traitement d'un cancer, ledit procédé comprenant l'administration à un individu atteint de cancer du virus oncolytique, de l'inhibiteur de point de contrôle immunitaire et de l'hydroxyurée, ledit virus oncolytique étant dérivé du virus de la vaccine, ledit virus de la vaccine étant un virus recombinant obtenu par délétion du gène de la thymidine kinase.

8. Virus oncolytique, inhibiteur de point de contrôle immunitaire et hydroxyurée destinés à être utilisés selon la revendication 7, ledit virus oncolytique, l'inhibiteur de point de contrôle immunitaire et l'hydroxyurée étant co-administrés simultanément, séquentiellement ou dans l'ordre inverse, ou
ladite hydroxyurée étant administrée avant, pendant ou après l'administration du virus oncolytique.

9. Virus oncolytique, inhibiteur de point de contrôle immunitaire et hydroxyurée destinés à être utilisés selon la revendication 7, ladite hydroxyurée étant administrée 3 à 5 jours avant l'administration du virus oncolytique et étant administrée en continu une fois par jour pendant 9 à 28 jours après l'administration du virus oncolytique.

10. Virus oncolytique, inhibiteur de point de contrôle immunitaire et hydroxyurée destinés à être utilisés selon la revendication 7, ledit inhibiteur de point de contrôle immunitaire étant administré après l'administration du virus oncolytique.

11. Virus oncolytique, inhibiteur de point de contrôle immunitaire et hydroxyurée destinés à être utilisés selon la revendication 7, ledit inhibiteur de point de contrôle immunitaire étant administré en continu au moins une fois par semaine pendant 1 semaine à 10 semaines après l'administration du virus oncolytique.

12. Virus oncolytique, inhibiteur de point de contrôle immunitaire et hydroxyurée destinés à être utilisés selon la revendication 7, ladite hydroxyurée étant administrée à une dose de 10 mg/kg/jour à 90 mg/kg/jour, ou ledit virus oncolytique étant administré à une dose de 1x10⁵ pfu à 1x10¹⁰ pfu.

13. Virus oncolytique, inhibiteur de point de contrôle immunitaire et hydroxyurée destinés à être utilisés selon la revendication 7, ledit virus oncolytique étant administré à l'individu à des intervalles de 7 à 30 jours.

14. Virus oncolytique, inhibiteur de point de contrôle immunitaire et hydroxyurée destinés à être utilisés selon la revendication 7, l'hydroxyurée étant administrée par voie intrapéritonéale ou intraveineuse, ou
ledit virus oncolytique étant administré par voie intratumorale, intrapéritonéale ou intraveineuse.
